# EUROPEAN PATENT SPECIFICATION

(11) **EP 3 192 497 B1**
(45) Date of publication and mention of the grant of the patent: **20.05.2020**
(21) Application number: 16150969.0
(22) Date of filing: 12.01.2016
(51) Int. Cl.: A61K 8/66, A61Q 11/00, A61K 8/73

(54) **ORAL CARE COMPOSITION COMPRISING AN ENZYME AND A NATURAL GUM**
MUNDPFLEGEZUSAMMENSETZUNG ENTHALTEND EIN ENZYM UND EINEN NATÜRLICHEN GUMMI
COMPOSITION DE PROTECTION ORALE CONTENANT UN ENZYME ET UNE GOMME NATURELLE

(43) Date of publication of application: 19.07.2017
(73) Proprietor: Unilever PLC, London, Greater London EC4Y 0DY (GB); Unilever N.V., 3013 AL Rotterdam (NL)
(72) Inventor: BARFOOT, Richard Jonathan, Merseyside CH63 3JW (GB); PERISSINOTO, Martina, Merseyside CH63 3JW (GB)
(74) Representative: Tansley, Sally Elizabeth

(56) References cited:
- EP-A1- 1 334 710
- FR-A1- 3 020 758
- JP-A- 2002 322 088
- US-A- 5 000 939
- US-A1- 2005 260 266
- OLSSON J ET AL: "Modulation of bacterial binding to salivary pellicle by treatment with hydrophilizing compounds", ARCHIVES OF ORAL BIOLOGY, PERGAMON PRESS, OXFORD, GB, vol. 35, 1 January 1990 (1990-01-01), pages S137-S140, XP027255160, ISSN: 0003-9969 [retrieved on 1990-01-01]

## Description

### Field of the Invention

The present invention is concerned with oral care compositions. More particularly, the present invention is concerned with oral care compositions that enhance and/or maintain the dental pellicle.

### Background of the Invention

The dental pellicle is a protein film that forms on the surface of teeth. It helps protect the teeth from external attacks, such as acid erosion and wear.

Compositions for applying onto the oral cavity for inducing saliva production are described in JP 2002 322088 , the compositions comprise a proteinic active ingredient selected from lactoferrin, lactoperoxidase, lysozyme

Glucose oxidase is present in compositions for use in the oral cavity including use as an adhesive for dentures as in EP1334710 or for tooth whitening as disclosed in US2005/260266.

US5000939 discloses oral compositions comprising carrageenan and dextranase.

FR 3020758 discloses compositions comprising an enzymatic complex comprising saccharase, -glucose oxidase, - amyloglucosidase, - lactoperoxidase, - lactoferrin. which is said to reinforce the bactericidal activity of saliva.

Olsson J et al (Archives of Oral Biology, Pergamon Press, Oxford, vol 35) discloses modulation of bacterial binding to the salivary pellicle by treatment with a polysachharide derivative such as a cellulose ether and a polyethylene glycol derivative.

The dental pellicle can be removed when the teeth are brushed. The present application relates to compositions that clean the teeth and help maintain the dental pellicle.

### Description of the Invention

The present invention relates to a composition comprising an enzyme and a natural gum for use in a method to maintain and/or enhance the mass of the salivary pellicle in which the enzyme consists of a mixture of amylglucosidase, glucose oxidase and lactoperoxidase and in which the natural gum is carrageenan.

### Detailed Description of the Invention

The present invention relates to a composition that deposits on the existing pellicle layer and thus helps protect the pellicle. It is thought that this deposition results in a change in the surface of the pellicle and helps build up an increased pellicle layer or enhance the layer. This increase in mass of the pellicle is, in the context of the present invention, seen as an enhancement.

The present invention relates to a method of maintaining and enhancing the salivary pellicle, of the teeth.

The composition of the invention comprises an enzyme, which consists of a mixture of amylglucosidase, glucose oxidase and lactoperoxidase.

Preferably the total level of enzyme is from 0.01 wt% to 2wt% of the total composition. More preferably from 0.1 wt% to 1 wt% of the total composition.

Preferably the total level of glycoside hydrolase in the composition is from 0.05 to 1 wt% of the total composition.

Preferably the total level of oxoreductase acting on OH groups of donors is from 0.05 to 1 wt% of the total composition.

Preferably the total level of hemeperoxidase is from 0.0005 to 0.01 wt% of the total composition. Preferably the weight ratio of glycoside hydrolase to other enzymes in the composition is greater than 1:1, more preferably greater than 3:2.

The oral care composition, also comprises the natural gum carrageenan.

A preferred carrageenan gum having has major amounts of kappa and lambda constituents. Carrageenan is a high molecular weight linear polysaccharide derived from sea plants which makes up approximately 2-7% of the plant and is found between the cellulosic fibres and is composed of repeating galactose units, and 3,6 anhydrogalactose (3,6-AG), both sulfated and nonsulfated, joined by alternating α 1-3, β 1-4 glycosidic linkages. A preferred carrageenan gum is available commercially from FMC Corporation under the Trademark Viscarin TP-206 which typically contains 62% lambda, 30% kappa and 8% iota forms.

Preferably the level of carrageenan is from 0.1 wt% to 1.5 wt% of the total composition, more preferably from 0.3 wt% to 1.0 wt%.

Compositions of the invention may also comprise gums such as xanthan gum

The total natural gums are present at levels from 0.1 wt% to 4 wt%, more preferably from 0.1 to 3 wt% of the total composition.

Additionally other structurant agents such as binders and thickening agents may be present. These structurants will generally be present in an amount of from 0.1 to 1 % by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose).

Compositions of the invention may also comprise other proteins such as lysozymes, lactoferrins and colostrum. Preferably these other proteins are present at total levels from 0.001 to 0.5 wt% of the total composition, preferably from 0.005 to 0.1 wt% of the total composition.

Furthermore, the oral care composition comprises a nonionic surfactant in an amount of from 0.2 to 5% by weight based on the total weight of the dentifrice. Preferred nonionic surfactants are polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Particularly preferred are polyethylene glycol ethers of fatty alcohols in particular polyethylene glycol ethers having from 20 to 40 ethylene oxide groups per unit. An example of such a suitable surfactant includes the group of surfactants known as Steareth surfactants such as Steareth 30. Preferably the level of non-ionic surfactant is from 0.5 to 7 wt% of the total composition, more preferably from 1 to 5 wt% of the total composition.

Although other surfactants may be present it is preferred if they are limited to levels below 0.5 wt% of the total composition preferably less than 0.1 wt% of the total composition. It is particularly preferred if the composition is substantially free of anionic surfactants that it that it comprises less than 0.05 wt% of anionic surfactant.

Examples of suitable product forms for compositions of the invention include dentifrices, mouthwashes, mouthsprays and liquid formulations.

Preferred product forms for compositions of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Glycerol and sorbitol are preferred, sorbitol being particularly preferred.

The oral care composition, in particular dentifrice compositions comprises abrasive materials. Abrasive materials will generally be present in an amount of from 0.5 to 75%, preferably 3 to 60 by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions of the invention may also comprise thickeners such as finely divided silicas, hectorites, calcium carbonates, colloidal magnesium aluminium silicates and mixtures thereof. Compositions of the present invention may also contain further optional ingredients customary in the art such as fluoride ion sources, anticalculus agents, buffers, flavouring agents, bleaching agents, sweetening agents, colouring agents, opacifying agents, preservatives, antisensitivity agents and antimicrobial agents.

Compositions of the invention may comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

The invention will now be illustrated by the following non-limiting Examples:

### Examples

**Example 1**

| **Material** | **Amount % W/W** |
|---|---|
| Sorbitol | 28.5 |
| Zinc Gluconate | 0.3 |
| Sodium Fluoride | 0.3 |
| Citric Acid | 0.3 |
| Disodium Phosphate | 0.5 |
| Sodium cCaseinate? | 0.01 |
| Titanium dioxide | 0.5 |
| Zeodent 113* | 16.0 |
| Tixosil 43** | 4.8 |
| Carrageenan | 0.7 |
| Glycerin | 5.0 |
| Xanthum gum | 1.0 |
| Steareth 30 | 3.0 |
| Amylglucosidase | 0.3 |
| Lactoperoxidase | 0.002 |
| Lysozyme (22%)*** | 0.05 |
| Glucose oxidase | 0.14 |
| Lactoferrin | 0.01 |
| Colostrum | 0.01 |
| Water and minors | to 100.0 |

| | |
|---|---|
| * Precipitated silica ** Thickening silica *** 0.0495 % lysozyme liquid at 22% lysozyme (containing 1450ppm F as NaF) | |

**Example A**

| **Material** | **Amount (%W/W)** |
|---|---|
| Sorbitol 70/70 | 45 |
| PEG-32 | 5 |
| Monosodium | 0.1 |
| Phosphate | |
| Sodium Fluoride | 0.32 |
| Titanium | 1 |
| Dioxide | |
| Tixosil 43* | 7.5 |
| Sorbosil AC77** | 10 |
| Celulose gum | 0.63 |
| Sodium Lauryl sulphate | 1.5 |
| | To 100 |

| | |
|---|---|
| Silica SLS Toothpaste (containing 1450ppm F as NaF) * hydrated thickening silica ** hydrated silica | |

### QCM-D of Salivary Pellicle

Quartz Crystal Microbalance with Dissipation (QCM-D) is a state of the art technique that allows nanoscale mass and structural changes to be measured. The measurements are based on changes in vibration frequency of a quartz crystal sensor in response to interaction (adsorption) and reactions at the sensor surface(www.biolinscientific.com/q-sense). QCM-D has been extensively applied to understand saliva pellicle on a range of substrates including the impact of detergents such as SLS (SDS) on the formed pellicle. It has been concluded that SLS elutes and decreases the mass of protein adsorbed in the pellicle layer.

QCM was used to measure the effects of Example A and Example 1 toothpaste on an in-vitro pellicle. In summary, treatment with a slurry of Example 1 toothpaste (1 part paste, 3 parts deionised water) maintains the pellicle mass in contrast to the Example A toothpaste slurry which shows a reduced pellicle mass.

## Claims

1. A composition comprising an enzyme and a natural gum for use in a method to maintain and/or enhance the mass of the salivary pellicle in which the enzyme consists of a mixture of amylglucosidase, glucose oxidase and lactoperoxidase and in which the natural gum is carrageenan.

2. A composition for use according to any preceding claim in which the pellicle is the salivary pellicle of the teeth.

3. A composition for use according to any preceding claim in which the composition further comprises a nonionic surfactant.

4. A composition for use according to claim 3 in which the non-ionic surfactant is a polyethylene glycol ether of a fatty alcohol.

5. A composition for use according to claim 4in which the non-ionic surfactant is Steareth 30.

6. A composition for use according to any preceding claim in which the composition comprises less than 0.05 wt% of anionic surfactant.

7. A composition for use according to any preceding claim in which the composition comprises a further protein.

8. A composition for use according to any preceding claim in which the composition is in the form of a dentifrice.

## Patentansprüche

1. Zusammensetzung, umfassend ein Enzym und einen natürlichen Gummi, zur Verwendung in einem Verfahren zum Aufrechterhalten und/oder zum Verbessern der Masse des Speichelpellikels, in welchem das Enzym aus einer Mischung von Amyloglucosidase, Glucose-Oxidase und Lactoperoxidase besteht, und in welcher der natürliche Gummi Carrageen ist.

2. Zusammensetzung zur Verwendung nach einem vorhergehenden Anspruch, in welchem das Pellikel das Speichelpellikel der Zähne ist.

3. Zusammensetzung zur Verwendung nach einem vorhergehenden Anspruch, in welcher die Zusammensetzung ferner nicht-ionisches Tensid umfasst.

4. Zusammensetzung zur Verwendung nach Anspruch 3, in welcher das nichtionische Tensid ein Polyethylenglycolether eines Fettalkohols ist.

5. Zusammensetzung zur Verwendung nach Anspruch 4, in welcher das nichtionische Tensid Steareth-30 ist.

6. Zusammensetzung zur Verwendung nach einem vorhergehenden Anspruch, in welcher die Zusammensetzung weniger als 0,05 Gew.-% anionisches Tensid umfasst.

7. Zusammensetzung zur Verwendung nach einem vorhergehenden Anspruch, in welcher die Zusammensetzung ein weiteres Protein umfasst.

8. Zusammensetzung zur Verwendung nach einem vorhergehenden Anspruch, in welcher die Zusammensetzung in Form eines Zahnputzmittels vorliegt.

## Revendications

1. Composition comprenant une enzyme et une gomme naturelle pour une utilisation dans un procédé pour maintenir et/ou promouvoir la masse de la pellicule salivaire dans laquelle l'enzyme consiste en un mélange d'amylglucosidase, glucose oxydase et lactoperoxydase et dans laquelle la gomme naturelle est le carraghénane.

2. Composition pour une utilisation selon l'une quelconque des revendications précédentes, dans laquelle la pellicule est la pellicule salivaire des dents.

3. Composition pour une utilisation selon l'une quelconque des revendications précédentes, dans laquelle la composition comprend de plus un tensioactif non ionique.

4. Composition pour une utilisation selon la revendication 3, dans laquelle le tensioactif non-ionique est un polyéthylène glycol éther d'un alcool gras.

5. Composition pour une utilisation selon la revendication 4, dans laquelle le tensioactif non-ionique est Steareth 30.

6. Composition pour une utilisation selon l'une quelconque des revendications précédentes, dans laquelle la composition comprend moins de 0,05 % en masse de tensioactif anionique.

7. Composition pour une utilisation selon l'une quelconque des revendications précédentes, dans laquelle la composition comprend une autre protéine.

8. Composition pour une utilisation selon l'une quelconque des revendications précédentes, dans laquelle la composition est dans la forme d'un dentifrice.
